# EUROPEAN PATENT SPECIFICATION

(11) **EP 2 005 887 B1**
(45) Date of publication and mention of the grant of the patent: **12.05.2010**
(21) Application number: 08010742.8
(22) Date of filing: 12.06.2008
(51) Int. Cl.: A61B 5/103

(54) **Gait assessment system**
Gangzugriffssystem
Système d'évaluation de la marche

(30) Priority: 23.06.2007 JP 2007165712
(43) Date of publication of application: 24.12.2008
(73) Proprietor: TANITA CORPORATION, Tokyo 174-8630 (JP)
(72) Inventor: Takehara, Katsumi, Tokyo 174-8630 (JP); Nishibayashi, Kenji, Tokyo 174-8630 (JP)
(74) Representative: Müller-Boré & Partner Patentanwälte

(56) References cited:
- EP-A- 1 066 793
- US-A- 5 485 402
- US-A1- 2002 040 601
- US-B1- 6 836 744

## Description

### Field of the Invention

The present invention relates to gait assessment systems and suchlike, which include a basograph equipped with a measuring instrument for measuring data related to gait of a subject, and a computer capable of receiving the gait-related data measured by the measuring instrument through a predetermined reception method.

### Description of the Related Art

Examples of muscles used for walking include quadriceps, which are used in, for example, lifting of the leg, anterior tibial muscles or tibialls anterior, which are used in, for example, landing support and lifting of the toes, and triceps surae, which are used in, for example, stepping, and lifting of the heel. Atrophy of these muscles due to aging results in shuffling, which can cause a fall. Conventionally, to prevent the elderly or the gait-impaired people from falling, trainers, such as physical therapists or occupational therapists, have provided various instructions in fall prevention classes for the elderly, for example. However, the teaching method varies from one trainer to another, and therefore in order to create an exact advisory training program, it is necessary to quantify the quality of gait. Accordingly, various devices have been developed to detect foot movement during walking.

An example of the aforementioned devices uses a pressure mat having measuring instruments such as sensors or the like laid in a walking path in order to detect foot movement during walking on the pressure mat (http://www.nitta.co.jp/images/product/pdf/tactile_product/gaitscan_pdf). Another exemplary device is of a motion capture type, which employs optical methodology in which a marker or suchlike is attached to the foot, and movement of the marker or suchlike is detected by cameras in a specialized studio set up in a dark place, for example.

Both the device that uses the pressure mat and the device of the motion capture type are large-sized devices, and therefore there was a problem that it is extremely expensive. Furthermore, the device that uses the pressure mat requires troublesome work, such as laying the pressure mat and extending required cables, and therefore there was a problem that it cannot be readily used by, for example, trainers. There was a problem that the device of the motion capture type requires a specialized measurement space.

EP 1 066 793 A2 and US 2002/0040601 disclose a motion analysis system. A device comprised of at least a pair of accelerometers and a tilt sensor mounted in fixed relation to a datum plane defining surface may be used for extracting kinematic variables including linear and rotational acceleration, velocity and position.

Further, a gait activity monitor is known from US 5,485,402 A. A monitor records the gait activity of a wearer. The monitor is constructed to determine and record the number of steps taken by a wearer during selected intervals. The monitor includes an optical transmitter receiver to permit the monitor to be optically coupled to a stand-alone computer for transmitting data therebetween. The gait activity data stored by the monitor can be downloaded to the stand-alone computer for analyzing the data and generating selected reports.

US 6,836,744 B1 discloses a portable system for analyzing human gait comprising of at least one independent rear foot motion collection unit, at least one independent lower shank motion collection unit, plantar pressure collection unit, at least one processing and display unit, and a soft casing unit. Further, a plurality of accelerometers, rate sensors, force sensor resistors, and pressure sensors provide for the acquisition of acceleration signals, angular velocity signals, foot force signals, and foot pressure signals to be processed.

### SUMMARY OF THE INVENTION

Therefore, the present invention has been made to solve problems as described above, and an objective thereof is to provide a compact and inexpensive gait assessment system, including a basograph, which improves the evaluation of the gait status.

According to the present invention, there is provided a gait assessment system comprising; a measuring instrument for measuring gait-related data of a subject, a basograph equipped with the measuring instrument, and a computer capable of receiving the gait-related data measured by the measuring instrument through a predetermined reception method, wherein the computer includes an estimating means for estimating a gait status of the subject based on the received gait-related data, wherein the computer further comprises an assessing means for calculating image data such that the gait status of the subject can be presented as an image in the form of a foot at each sampling time, based on estimation by the estimating means, and a presentation means for presenting a visualized gait of the subject on the ground or floor based on the image data calculated by the assessing means.

The basograph may include an acceleration sensor as a part of the measuring instrument, the acceleration sensor measuring, as the gait-related data, predetermined direction acceleration of the subject's foot at each predetermined time, and the estimating means may include at least one of the following means for performing estimation based on the predetermined-direction acceleration of the subject's foot at each predetermined time: a push-off estimating means for estimating the intensity of the subject's gait tempo, an elevation estimating means for estimating elevation of the subject's foot, and a stride estimating means for estimating the subject's stride.

In preferred embodiment, the basograph further includes an inclination sensor as a part of the measuring instrument, the inclination sensor measuring, as the gait-related data, a predetermined-direction inclination of the subject's foot at each predetermined time, and the estimating means further includes a landing estimating means for estimating a landing status of the subject's foot based on the predetermined-direction acceleration of the subject's foot at each predetermined time, and the predetermined-direction inclination of the subject's foot at each predetermined time.

In another preferred embodiment, the basograph further includes an angular velocity sensor as a part of measuring instrument, the angular velocity sensor measuring, as the gait-related data, an angular velocity of the subject's foot at each predetermined time, and the estimating means further includes a moving-status estimating means for estimating a moving status of the subject based on the predetermined-direction acceleration of the subject's foot at each predetermined time, and the angular velocity of the subject's foot at each predetermined time.

Further, the basograph may also include an operating means for performing a predetermined operation based on the gait-related data measured by the measuring instrument.

The above and other objects, effects, features and advantages of the present invention will become more apparent from the following description of the embodiments thereof taken in conjunction with the accompanying drawings.

### BRIEF DESCRIPTION OF THE DRAWINGS

Fig. 1 illustrates a gait assessment system 1 according to a first preferred embodiment of the present invention.
Fig. 2 is a graph for describing the relationship between movement of the foot 40 and the acceleration ACC.
Fig. 3 illustrates an example where the presentation section 31 presents the assessment result by the assessing section 30 on the presentation device 35, such as a display.
Fig. 4 illustrates another example where the presentation section 31 presents the assessment result by the assessing section 30 on the presentation device 35, such as a display.
Fig. 5 shows, in flowchart form, the functional flow of a gait assessment program of the present invention.
Fig. 6 is a block diagram illustrating an internal circuit 60 of the PC 20 that executes a computer program of the present invention to realize each of the above embodiments.

### DESCRIPTION OF THE PREFERRED EMBODIMENTS

Various embodiments of the present invention will be described herein below with reference to the accompanying drawings. It is to be noted that the same or similar reference numerals are applied to the same or similar parts and elements throughout the drawings, and the description of the same or similar parts and elements will be omitted or simplified.

### First Preferred Embodiment

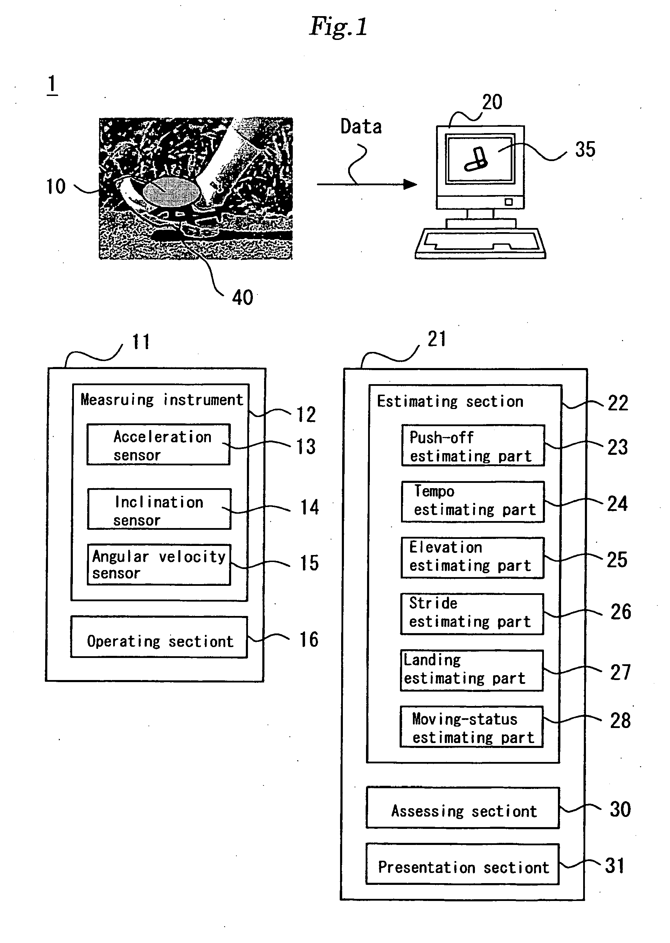

Fig. 1 illustrates a gait assessment system 1 according to a first preferred embodiment of the present invention. In Fig. 1, reference numeral 40 denotes a foot of a subject; 10, a basograph attached to the subject's foot 40; 20, a personal computer (PC) for receiving data related to gait of the subject and performing various operations, such as estimation and assessment; and 35, a presentation device, such as a display of the PC 20. The foot 40 is preferably an instep, as shown in Fig. 1. Note that, in some cases, the term "foot" as used herein refers not only to the foot but also to the ankle, the lower leg, etc. Attaching only one basograph 10 to the subject's foot (dominant foot) is sufficient, but the basograph 10 may be attached to each foot. The basograph 10 is equipped with a measuring instrument 12 for measuring the gait-related data of the subject, as shown within an internal block 11 of the basograph 10 in Fig. 1. The PC 20 is capable of receiving the gait-related data measured by the measuring instrument 12 through a predetermined reception method. In a preferred reception method, for example, the gait-related data (DATA) is transmitted from the basograph 10 to the PC 20 via a wired connection, such as a USB (universal serial bus) interface between the basograph 10 and the PC 20. The predetermined reception method may be wireless reception. The PC 20 includes an estimating section 22 for estimating the gait status of the subject based on the received gait-related data (DATA), as shown within a functional block 21 indicating functions of the PC 20 in Fig. 1.

Described below are several parts of the measuring instrument 12, and estimation performed by the estimating section 22 based on the gait-related data (DATA) measured by the measuring instrument 12. The basograph 10 includes an acceleration sensor 13 as a part of the measuring instrument 12, as shown within the internal block 11 in Fig. 1. The acceleration sensor 13 is preferably a three-dimensional acceleration sensor produced by MEMS (Micro-Electro-Mechanical Systems) technology. In the case of using the acceleration sensor 13, the gait-related data (DATA) contains predetermined-direction acceleration ACC of the subject's foot 40 measured by the acceleration sensor 13 at each sampling time (predetermined time). The estimating section 22 includes at least one of the following elements for performing estimation based on the predetermined-direction acceleration ACC of the subject's foot 40 at each sampling time: a push-off estimating part (push-off estimating means) 23 for estimating the intensity of the subject's push-off; a tempo estimating part (tempo estimating means) 24 for estimating gait tempo; an elevation estimating part (elevation estimating means) 25 for estimating elevation of the foot; and a stride estimating part (stride estimating means) 26.

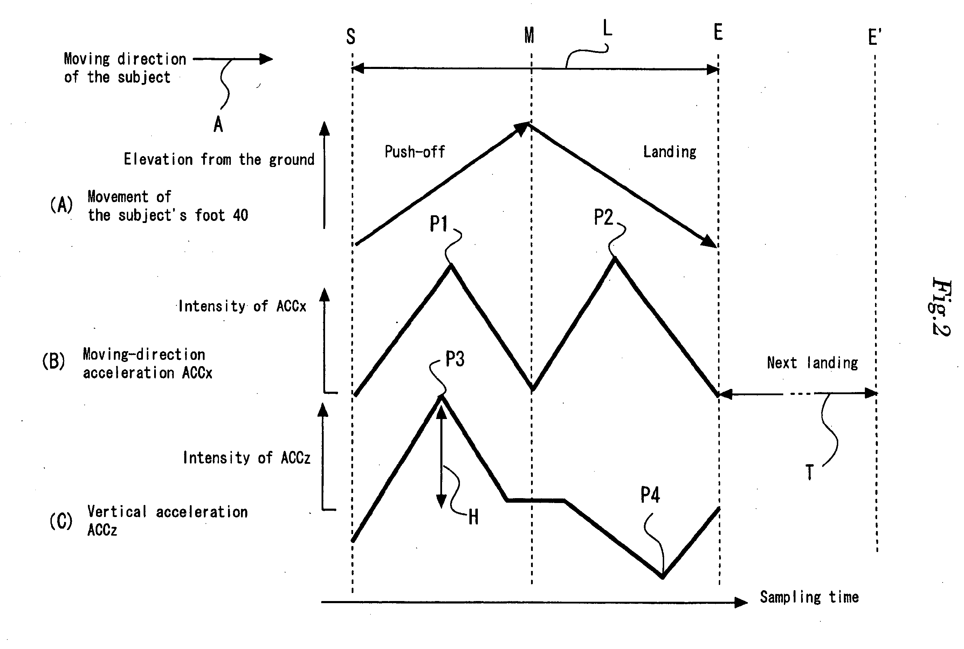

The relationship between movement of the foot 40 and the acceleration ACC will now be briefly described. Fig. 2 is a graph for describing the relationship between movement of the foot 40 and the acceleration ACC. The moving direction of the subject is a direction indicated by arrow A shown in Fig. 2. In Fig. 2A, movement of the subject's foot 40 is shown, for which the vertical axis indicates the elevation from the ground, and the horizontal axis indicates the sampling time. In Fig. 2B, variations of moving-direction acceleration ACCx in accordance with the movement of the subject's foot 40 are shown, for which the vertical axis indicates the intensity of the moving-direction acceleration ACCx, and the horizontal axis indicates the sampling time. In Fig. 2C, variations of vertical acceleration ACCz in accordance with the movement of the subject's foot 40 are shown, for which the vertical axis indicates the intensity of the vertical acceleration ACCz, and the horizontal axis indicates the sampling time. The predetermined-direction acceleration ACC includes both the moving-direction acceleration ACCx and the vertical acceleration ACCz, but in some cases, it may be either one of them.

As shown in Fig. 2A, the foot 40 positioned behind the body starts moving by push-off at a sampling time denoted by "S", and then the foot 40 is raised toward the body and elevated to its maximum height at a sampling time denoted by "M" before moving ahead of the body to land on the ground at a sampling time denoted by "E". As shown in Fig. 2B, the moving-direction acceleration ACCx starts to increase from 0 at the sampling time "S" because of the movement by push-off, and reaches peak P1 between "S" and "M". Thereafter, as the foot 40 is raised toward the body, the moving-direction acceleration ACCx starts to decrease with the velocity of the foot 40, and it falls to 0 at the sampling time "M" when the foot 40 is elevated to the maximum height toward the body. Subsequently, the moving-direction acceleration ACCx starts to increase as the foot 40 moves ahead of the body, and reaches peak P2 between "M" and "E". Thereafter, as the foot 40 moves closer to the ground for landing, the moving-direction acceleration ACCx starts to decrease with the velocity of the foot 40, and it falls to 0 at the sampling time "E" when the foot 40 lands on the ground. As shown in Fig. 2C, the vertical acceleration ACCz starts to increase from 0 toward the positive side at the sampling time "S" because of push-off, and reaches peak P3 between "S" and "M". Thereafter, as the foot 40 is raised toward the body, the vertical acceleration ACCz starts to decrease with the velocity of the foot 40, and it falls to 0 at the sampling time "M" when the foot 40 is elevated to the maximum height toward the body. Subsequently, the vertical acceleration ACCz starts to increase toward the negative side as the foot 40 moves down, and reaches negative peak P4 between "M" and "E". Thereafter, as the foot 40 moves closer to the ground for landing, the vertical acceleration ACCz starts to decrease with the velocity of the foot 40, and it falls to 0 at the sampling time "E" when the foot 40 lands on the ground. Each estimating part, such as 23, of the estimating section 22 can obtain both the start time of gait (i.e., the sampling time "S"; hereinafter, referred to as the "gait start time S") and the end time of gait (i.e., the sampling time "E"; hereinafter referred to as the "gait end time E") based on the moving-direction acceleration ACCx (the predetermined-direction acceleration) of the subject's foot 40 at each sampling time.

The push-off estimating part 23 is capable of estimating the intensity of the subject's push-off based on the intensity of the vertical acceleration ACCx (= 0) at the gait start time S shown in Fig. 2C, as well as the sampling time and the intensity of the vertical acceleration ACCz (= H) at peak P3. As described above, the gait start time S can be obtained based on the moving-direction acceleration ACCx of the subject's foot 40 at each sampling time.

The tempo estimating part 24 is capable of estimating the gait tempo based on a period T from the gait end time E in Fig. 2A to a sampling time for the next landing denoted by "E"'. As described above, landing can be obtained based on the moving-direction acceleration ACCx of the subject's foot 40 at each sampling time. It is also possible to estimate the number of steps based on the period T.

The elevation of the foot 40 can be measured, for example, by using an altitude sensor (not shown) including an ultrasonic sensor or suchlike. Alternatively, the elevation estimating part 25 is capable of obtaining the average vertical velocity of the foot 40 based on the intensity H of the vertical acceleration ACCz at peak P3 shown in Fig. 2C and a period (t) for the foot 40 to reach peak P3, thereby estimating the elevation of the foot 40 based on the average velocity and the period t.

The stride estimating part 26 is capable of obtaining the average moving-direction velocity of the foot 40 based on the moving-direction acceleration ACCx between the gait start time S and the gait end time E shown in Fig. 2B, and a period L (gait period), which is obtained by subtracting the start time S from the end time E, thereby estimating the stride, i.e., the moving distance of the foot 40, based on the average velocity and the period L. As described above, the gait start time S and the gait end time E can be obtained based on the moving-direction acceleration ACCx of the subject's foot 40 at each sampling time.

The basograph 10 can further include an inclination sensor 14 as a part of the measuring instrument 12. The inclination sensor 14 is preferably a small-sized, lightweight sensor. In the case of using the inclination sensor 14, the gait-related data (DATA) additionally contains a vertical (predetermined-direction) inclination CLI of the subject's foot 40 measured by the inclination sensor 14 at each sampling time. The estimating section 22 can further include a landing estimating part (landing estimating means) 27 for estimating the landing status of the subject's foot 40 based on the predetermined-direction acceleration ACC of the subject's foot 40 at each sampling time, and the predetermined-direction inclination CLI of the subject's foot 40 at each sampling time. For example, the landing status estimated by the landing estimating part 27 indicates whether the landing was made with the heel. As described above, the landing can be obtained based on the moving-direction acceleration ACCx of the subject's foot 40 at each sampling time. The landing estimating part 27 is capable of estimating whether the landing was made with the heel based on the vertical inclination CLI of the foot 40 measured by the inclination sensor 14 at the time of landing. For example, in the case where the vertical inclination CLI is 0° when the foot 40 is horizontal in the moving direction A shown in Fig. 2, the vertical inclination CLI is about +30° when the landing was made with the heel, and about -30° when the landing was made with the toes due to shuffling or suchlike. In this manner, the landing estimating part 27 can estimate whether the landing was made with the heel based on the sign (positive or negative) and the angle of the vertical inclination CLI.

The basograph 10 can further include an angular velocity sensor 15 as a part of the measuring instrument 12. The angular velocity sensor 15 is preferably a small-sized, one-chip-encapsulation-type angular velocity sensor or suchlike. In the case of using the angular velocity sensor 15, the gait-related data (DATA) additionally contains angular velocity ANG of the subject's foot 40 measured by the angular velocity sensor 15 at each sampling time. The estimating section 22 can further include a moving-status estimating part (moving-status estimating means) 28 for estimating the moving status of the subject based on the predetermined-direction acceleration ACC of the subject's foot 40 at each sampling time and the angular velocity ANG of the subject's foot at each sampling time. For example, the moving status estimated by the moving-status estimating part 28 indicates whether the subject exhibits staggering gait. As described above, by using a three-dimensional acceleration sensor as the acceleration sensor 13, it becomes possible to estimate the moving status of the subject on a plane in the moving direction. In addition, by using the angular velocity sensor 15 along with the acceleration sensor 13, it becomes possible to obtain the turning status of the foot 40, thereby estimating whether the subject exhibits staggering gait.

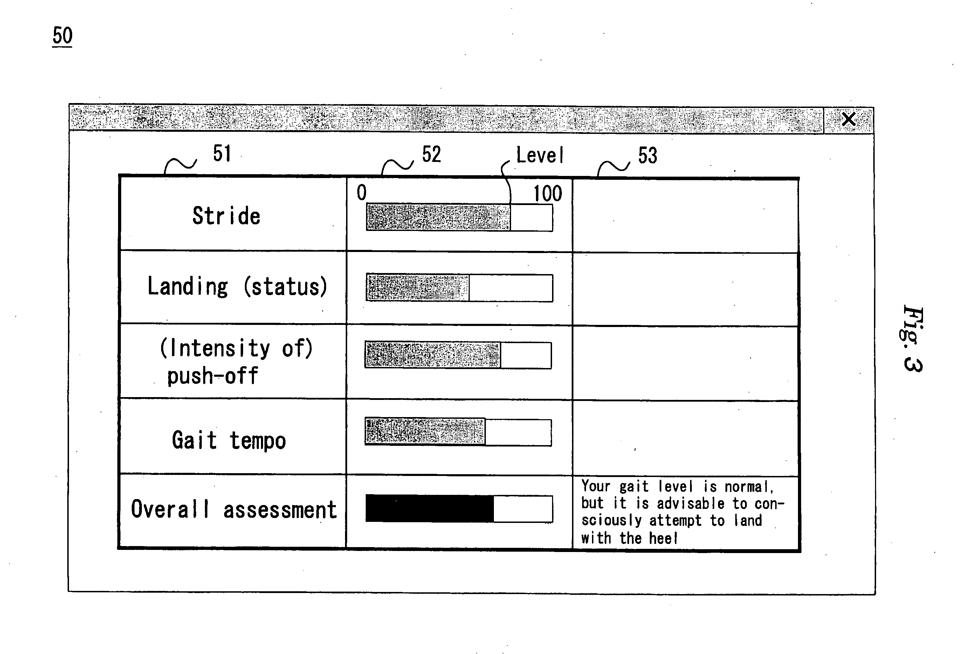

The PC 20 further includes an assessing section (assessing means) 30 for assessing the gait status of the subject based on estimation by the estimating section 22, and a presentation section (presentation means) 31 for presenting the assessment result by the assessing section 30 in a predetermined form. Fig. 3 illustrates an example where the presentation section 31 presents the assessment result by the assessing section 30 on the presentation device 35, such as a display. For example, the predetermined form is a tabular form 50 as shown in Fig. 3 to be described below. In Fig. 3, numeral 51 denotes a column for various gait status parameters estimated by the estimating section 22, which are, from top, the stride of the subject estimated by the stride estimating part 26, the landing (status) of the subject's foot estimated by the landing estimating part 27, (the intensity of) the subject's push-off estimated by the push-off estimating part 23, and the gait tempo of the subject estimated by the tempo estimating part 24, with overall assessment by the assessing section 30 being indicated at the bottom. Numeral 52 denotes an assessment column for assessments by the assessing section 30 concerning the gait status of the subject as indicated by level (Level) on a percentage scale from 0 to 100%, as shown in Fig. 3. Concretely, the averages for the gait status parameters, such as stride, among people of the same sex and age or generation as the subject is 50%, and the assessing section 30 indicates the gait status parameters, such as stride, of the subject by level (in percentages). The assessing section 30 indicates the overall assessment result for various gait status parameters at the bottom of the assessment column 52. Concretely, the average or weighted average of the levels of various gait status parameters is indicated. Numeral 53 denotes a column for comments added to the assessments indicated by level in the assessment column 52 by the assessing section 30. For simplification, Fig. 3 shows only the comment for the overall assessment. In Fig. 3, all the gait status parameters of the subject are above average, excepting the landing status, so that there is added a comment "Your gait level is normal, but it is advisable to consciously attempt to land with the heel.". In this manner, the assessing section 30 assesses various gait status parameters of the subject by level based on estimation by the estimating section 22 with reference to the averages for the gait status parameters, such as stride, classified by sex, age, and generation and previously recorded on the PC 20. Furthermore, the assessing section 30 makes suitable selections from among comments previously recorded in the PC 20 in accordance with the levels. The presentation section 31 presents the assessments (by level) and the comments by the assessing section 30 in the tabular form 50 as shown in Fig. 3 on the presentation device 35, such as a display.

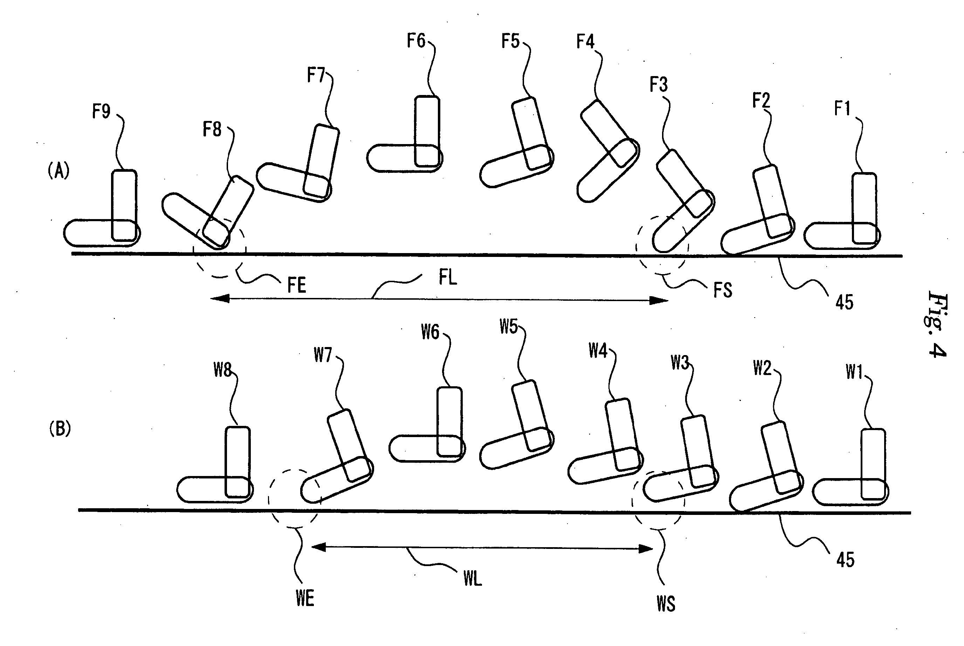

Fig. 4 illustrates another example where the presentation section 31 presents the assessment result by the assessing section 30 on the presentation device 35, such as a display. Fig. 4A indicates an exemplary subject with correct gait status, and Fig. 4B indicates an exemplary subject with shuffling gait status, in both of which the gait direction is from right to left. In Fig. 4A, numeral 45 denotes the ground or floor, "F1" through "F9" denote the subject's foot 40 at each sampling time, "FS" denotes takeoff, "FE" denotes landing, and "FL" denotes stride. In Fig. 4B, numeral 45 denotes the ground or floor as in Fig. 4A, "W1" through "W8" denote the subject's foot 40 at each sampling time, "WS" denotes takeoff, "WE" denotes landing, and "WL" denotes stride. The assessing section 30 is capable of calculating image data (making assessments) such that the gait status of the subject can be presented as an image in the form of a foot at each sampling time, based on estimation by the estimating section 22. The presentation section 31 is capable of presenting the image data (assessment result) obtained by the assessing section 30, as shown in Fig. 4A and Fig. 4B. That is, with the assessing section 30 and the presentation section 31, it is possible to visualize the gait of the subject. When comparing Fig. 4A and Fig. 4B, the takeoff WS of the subject with shuffling gait status is shown at a lower angle than the takeoff FS of the subject with correct gait status, indicating that the takeoff WS is weaker. The landing WE of the subject with shuffling gait status is shown to be made with the toes, whereas the landing FE of the subject with correct gait status is shown to be made with the heel. The stride WL of the subject with shuffling gait status is shown to be shorter than the stride FL of the subject with correct gait status. While the gait status of the subject in Fig. 4 is shown two-dimensionally, it can be shown three-dimensionally. In addition, while two subjects are compared in Fig. 4, three or more subjects can be compared.

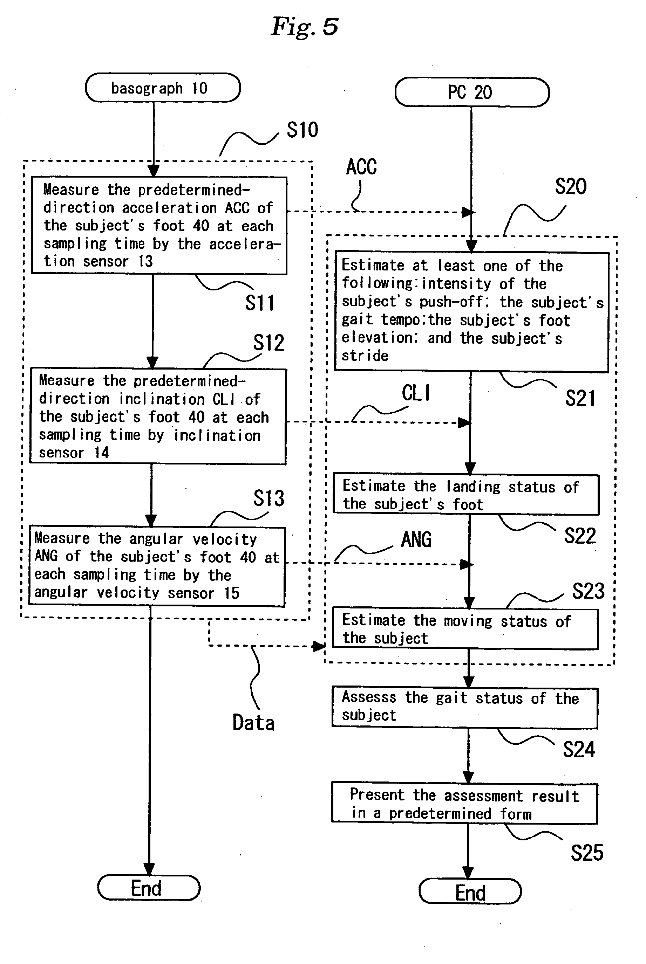

Fig. 5 shows, in flowchart form, the functional flow of a gait assessment computer program of the present invention. In Fig. 5, the left-side flowchart shows the function of the basograph 10, the right-side flowchart shows the function of the PC 20, and transmission from the basograph 10 to the PC 20 is indicated by dotted lines. First, the outline of the functional flow is described. As shown in Fig. 5, the basograph 10 measures the gait-related data (DATA) of the subject with the measuring instrument 12, and transmits the measured gait-related data (DATA) to the PC 20 via the aforementioned USB interface or suchlike (step S10). The estimating section 22 of the PC 20 estimates the gait status of the subject based on the transmitted gait-related data (DATA) (step S20). Thereafter, the assessing section 30 assesses the gait status of the subject (step S24), and the presentation section 31 presents the assessment result (step S25).

Next, the details of the functional flow are described. First, the acceleration sensor 13 measures the predetermined-direction acceleration ACC of the subject's foot 40 at each sampling time. The acceleration ACC at each sampling time is transmitted to the PC 20 via the USB interface or suchlike (thus far corresponding to step S11). In the PC 20, at least one of the following estimations is executed based on the transmitted acceleration ACC at each sampling time (step S21): estimation by the push-off estimating part 23, regarding the intensity of the subject's push-off estimation by the tempo estimating part 24, regarding the subject's gait tempo; estimation by the elevation estimating part 25, regarding the subject's foot elevation; and estimation by the stride estimating part 26, regarding the subject's stride.

Furthermore, the inclination sensor 14 measures the predetermined-direction inclination CLI of the subject's foot 40 at each sampling time. The inclination CLI at each sampling time is transmitted to the PC 20 via the USB interface or suchlike (thus far corresponding to step S12). The landing estimating part 27 estimates the landing status of the subject's foot based on the transmitted acceleration ACC at each sampling time and the transmitted inclination CLI at each sampling time (step S22).

Furthermore, the angular velocity sensor 15 measures the angular velocity ANG of the subject's foot 40 at each sampling time. The angular velocity ANG at each sampling time is transmitted to the PC 20 via the USB interface or suchlike (thus far corresponding to step S13). The moving-status estimating part 28 estimates the moving status of the subject based on the transmitted acceleration ACC at each sampling time and the transmitted angular velocity ANG at each sampling time (step S23).

The assessing section 30 assesses the gait status of the subject based on the above-described estimations obtained in steps S21 through S23 (step S24). The presentation section 31 presents the assessment result obtained in step S24 in a predetermined form (step S25).

As described above, in a first preferred embodiment of the present invention, the basograph 10 is equipped with the measuring instrument 12 for measuring the gait-related data of the subject. The PC 20 receives the gait-related data (DATA) measured by the measuring instrument 12 via the USB interface or suchlike. The estimating section 22 of the PC 20 estimates the gait status of the subject based on the received gait-related data (DATA). The basograph 10 includes the acceleration sensor 13 as a part of the measuring instrument 12. The acceleration sensor 13 is preferably a three-dimensional acceleration sensor produced by MEMS technology. The basograph 10 can further include the inclination sensor 14 as a part of the measuring instrument 12. The inclination sensor 14 is preferably a small-sized, lightweight sensor. The basograph 10 can further include the angular velocity sensor 15 as a part of the measuring instrument 12. The angular velocity sensor 15 is preferably a small-sized, one-chip-encapsulation-type angular velocity sensor or suchlike. As a result, the basograph 10 can be rendered compact in size, and provided at low cost. The basograph 10 can be simply attached to the foot 40 of the subject to detect movement of the foot during walking, thereby measuring the gait-related data of the subject, and therefore it can be readily handled by the trainer and the subject, eliminating the necessity to select any specialized measurement space, and allowing measurements to be taken in everyday life of the subject.

The estimating section 22 includes at least one of the following elements for performing estimation based on the predetermined-direction acceleration ACC of the subject's foot 40 at each sampling time: the push-off estimating part 23 for estimating the intensity of the subject's push-off; the tempo estimating part 24 for estimating the subject's gait tempo; the elevation estimating part 25 for estimating the elevation of the subject's foot; and the stride estimating part 26 for estimating the subject's stride. The estimating section 22 can further include the landing estimating part 27 for estimating the landing status of the subject's foot 40 based on the predetermined-direction acceleration ACC of the subject's foot 40 at each sampling time and the predetermined-direction inclination CLI of the subject's foot 40 at each sampling time. The estimating section 22 can further include the moving-status estimating part 28 for estimating the moving status of the subject based on the predetermined-direction acceleration ACC of the subject's foot 40 at each sampling time and the angular velocity ANG of the subject's foot 40 at each sampling time. The PC 20 further includes the assessing section 30 for assessing the gait status of the subject based on the estimation by the estimating section 22, and the presentation section 31 for presenting the assessment result by the assessing section 30 in a predetermined form. The assessing section 30 assesses various gait status parameters of the subject by level based on the estimation by the estimating section 22 with reference to the averages for the gait status parameters, such as stride, classified by sex, age, and generation previously recorded on the PC 20. Furthermore, the assessing section 30 makes suitable selections from among comments previously recorded in the PC 20 in accordance with the levels. The presentation section 31 could present the assessments (by level) and the comments by the assessing section 30 in the tabular form 50 on the presentation device 35, such as a display. According to the invention, with the assessing section 30 and the presentation section 31, it is possible to visually present the gait of the subject. In such a manner, the gait assessment system 1 can automatically provide a visual presentation of various assessments based on the gait-related data (DATA) measured by the basograph 10, and therefore, for example, the trainer can readily handle the gait assessment system 1. As a result, for example, the trainer can readily recognize the gait status of the subject, and can readily carry out screening for gait training of, for example, the elderly, making it possible to clarify teaching content.

### Second Preferred Embodiment

The basograph 10 can further include an operating section (operating means) 16 for performing a predetermined operation based on the gait-related data (DATA) measured by the measuring instrument 12. Examples of the operating section 16 include devices for performing, for example, an operation to produce vibration, an operation to output audio, such as buzzing sound, or an operation to emit light, or a device for performing a combination thereof. For example, when the subject is detected to be in the shuffling status based on the gait-related data (DATA) measured by the measuring instrument 12 (the acceleration ACC measured by the acceleration sensor 13 and the inclination CLI measured by the inclination sensor 14), the basograph 10 can perform, for example, an operation to produce vibration. The detection may be realized by incorporating, for example, the above-described function of the landing estimating part 27 into the basograph 10. Thus, when wearing the basograph 10, the subject can readily recognize his/her gait status in everyday life.

### Third Preferred Embodiment

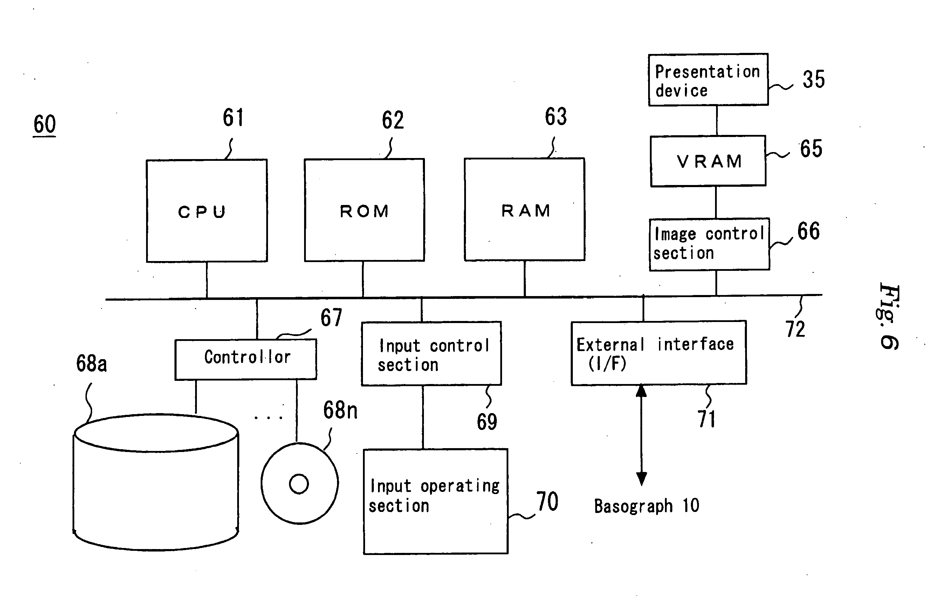

Fig. 6 is a block diagram illustrating an internal circuit 60 of the PC 20 that executes a computer program of the present invention to realize each of the above embodiments. A CPU 61, a ROM 62, a RAM 63, an image control section 66, a controller 67, an input control section 69, and an external interface (I/F) section 71 are connected to a bus 72, as shown in Fig. 6. In Fig. 6, the computer program of the present invention is recorded on a recording medium (including a removable recording medium), such as the ROM 62, a disk 68a, or a CD-ROM68n. For example, the disk 68a can have recorded thereon average gait status parameters, such as stride, classified by sex, age and generation for use in the assessing section 30, along with comments corresponding to levels. The computer program of the present invention is loaded onto the RAM 63 from the ROM 62 through the bus 72, or from the recording medium, such as the disk 68a or the CD-ROM68n, through the controller 67 and then through the bus 72. The input control section 69 is connected to an input operating section 70, such as a mouse or a numerical keypad, to perform input control, for example. A VRAM 65, which is image memory, has a capacity corresponding to data of at least one screen image of the presentation device 35, such as a display, and the image control section 66 functions to send the data on the VRAM 65 to the presentation device 35 after converting it into image data. The external I/F section 71 functions as, for example, an USB input/output interface with the basograph 10.

As described above, the CPU 61 executes the computer program of the present invention, thereby making it possible to attain the objective of the present invention. As described above, the computer program can be supplied to the CPU 61 of the PC 20 in the form of a recording medium, such as the CD-ROM 68n, so that the recording medium, such as the CD-ROM 68n, having the computer program recorded thereon constitutes the present invention as well. In addition to the above-described recording medium, for example, a memory card, a memory stick, a DVD, an optical disk, an FD, or the like, can be used for recording the computer program.

### Industrial Applicability

Application examples of the present invention include the cases where trainers, such as physical therapists or occupational therapists, provide various instructions in fall prevention classes for the elderly, for example.

In the gait assessment system of the present invention, the basograph is equipped with a measuring instrument for measuring data related to gait of a subject. A personal computer (PC) receives the gait-related data measured by the measuring instrument via a USB interface or suchlike. The estimating section of the PC estimates the gait status of the subject based on the received gait-related data. The basograph includes a small-sized acceleration sensor as a part of the measuring instrument. The basograph can further include a small-sized, lightweight inclination sensor as a part of the measuring instrument. The basograph can further include a small-sized, one-chip-encapsulation-type angular velocity sensor as a part of the measuring instrument. As a result, the basograph can be rendered compact in size, and provided at low cost. The basograph can be simply attached to the subject's foot to measure the gait-related data of the subject, and therefore it can be readily handled by the trainer and the subject, eliminating the necessity to select any specialized measurement space, and allowing measurements to be taken in everyday life of the subject. The estimating section includes at least one of the following elements for performing estimation based on predetermined-direction acceleration of the subject's foot at each sampling time: the push-off estimating part for estimating the intensity of the subject's push-off; the tempo estimating part for estimating gait tempo; the elevation estimating part for estimating elevation of the foot; and the stride estimating part. The estimating section can further include the landing estimating part for estimating the landing status of the subject's foot based on the predetermined-direction acceleration of the subject's foot at each sampling time and predetermined-direction inclination of the subject's foot at each sampling time. The estimating section can further include the moving-status estimating part for estimating the moving status of the subject based on the predetermined-direction acceleration of the subject's foot at each sampling time and angular velocity of the subject's foot at each sampling time. The PC further includes the assessing section for assessing the gait status of the subject based on the estimation by the estimating part, and a presentation section for presenting assessment results by the assessing section in a predetermined form. With the assessing section and the presentation section, it is possible to visually present the gait of the subject. Thus, the gait assessment system is capable of automatically providing a visual presentation of various assessments based on the gait-related data measured by the basograph, so that, for example, the trainer can readily handle the gait assessment system. As a result, for example, the trainer can readily recognize the gait status of the subject, and can readily carry out screening for gait training of, for example, the elderly, making it possible to clarify teaching content.

In the gait assessment system, the basograph may include an acceleration sensor as a part of the measuring instrument, the acceleration sensor measuring, as the gait-related data, predetermined-direction acceleration of the subject's foot at each predetermined time, and the estimating means includes at least one of the following means for performing estimation based on the predetermined-direction acceleration of the subject's foot at each predetermined time: a push-off estimating means for estimating the intensity of the subject's push-off; a tempo estimating means for estimating the subject's gait tempo; an elevation estimating means for estimating elevation of the subject's foot; and a stride estimating means for estimating the subject's stride.

In the gait assessment system, the basograph may further include an inclination sensor as a part of the measuring instrument, the inclination sensor measuring, as the gait-related data, a predetermined-direction inclination of the subject's foot at each predetermined time, and the estimating means further includes a landing estimating means for estimating a landing status of the subject's foot based on the predetermined-direction acceleration of the subject's foot at each predetermined time, and the predetermined-direction inclination of the subject's foot at each predetermined time.

In the gait assessment system, the basograph may further include an angular velocity sensor as a part of measuring instrument, the angular velocity sensor measuring, as the gait-related data, an angular velocity of the subject's foot at each predetermined time, and the estimating means further includes a moving-status estimating means for estimating a moving status of the subject based on the predetermined-direction acceleration of the subject's foot at each predetermined time, and the angular velocity of the subject's foot at each predetermined time.

In the gait assessment system, the basograph further includes an operating means for performing a predetermined operation based on the gait-related data measured by the measuring instrument.

Here, the gait assessment system may further comprise an assessing means for assessing the gait status of the subject based on the estimation by the estimating means.

Here, the gait assessment system may further comprise a presentation means for presenting an assessment result by the assessing means in a predetermined form.

Here, the basograph may further comprise an acceleration sensor as a part of the measuring instrument, the acceleration sensor measuring, as the gait-related data, predetermined-direction acceleration of the subject's foot at each predetermined time, based on the predetermined-direction acceleration of the subject's foot at each predetermined time, the computer may estimate at least one of the following: the intensity of the subject's push-off the subject's gait tempo; elevation of the subject's foot; and the subject's stride.

Here, the basograph may further comprise an inclination sensor as a part of the measuring instrument, the inclination sensor measuring, as the gait-related data, a predetermined-direction inclination of the subject's foot at each predetermined time, the computer may further estimate a landing status of the subject's foot based on the predetermined-direction acceleration of the subject's foot at each predetermined time, and the predetermined-direction inclination of the subject's foot at each predetermined time.

Here, the basograph may further comprise an angular velocity sensor as a part of the measuring instrument, the angular velocity sensor measuring, as the gait-related data, an angular velocity of the subject's foot at each predetermined time, the computer may further estimate a moving status of the subject based on the predetermined-direction acceleration of the subject's foot at each predetermined time, and the angular velocity of the subject's foot at each predetermined time.

Here, the basograph may further comprise an operating means for performing a predetermined operation based on the gait-related data measured by the measuring instrument.

In the gait assessment program, the basograph may include an acceleration sensor as a part of the measuring instrument, the acceleration sensor measuring, as the gait-related data, predetermined-direction acceleration of the subject's foot at each predetermined time, and the estimating means may include at least one of the following means for performing estimation based on the predetermined-direction acceleration of the subject's foot at each predetermined time: a push-off estimating means for estimating the intensity of the subject's push-off, a tempo estimating means for estimating the subject's gait tempo; an elevation estimating means for estimating elevation of the subject's foot; and a stride estimating means for estimating the subject's stride.

In the gait assessment program, the basograph may further include an inclination sensor as a part of the measuring instrument, the inclination sensor measuring, as the gait-related data, a predetermined-direction inclination of the subject's foot at each predetermined time, and the estimating means may further include a landing estimating means for estimating a landing status of the subject's foot based on the predetermined-direction acceleration of the subject's foot at each predetermined time, and the predetermined-direction inclination of the subject's foot at each predetermined time.

In the gait assessment program, the basograph may further include an angular velocity sensor as a part of the measuring instrument, the angular velocity sensor measuring, as the gait-related data, an angular velocity of the subject's foot at each predetermined time, and the estimating means may further include a moving-status estimating means for estimating a moving status of the subject based on the predetermined-direction acceleration of the subject's foot at each predetermined time, and the angular velocity of the subject's foot at each predetermined time.

Here, the gait assessment program may further comprise an assessing means for assessing the gait status of the subject based on the estimation by the estimating means.

Here, the gait assessment program may further comprise a presentation means for presenting an assessment result by the assessing means in a predetermined form.

## Claims

1. A gait assessment system comprising.
a measuring instrument (12) for measuring gait-related data of a subject, a basograph (10) equipped with the measuring instrument, and a computer (20) capable of receiving the gait-related data measured by the measuring instrument through a predetermined reception method,
wherein said computer (20) includes:
an estimating means (22) for estimating a gait status of the subject based on the received gait-related data,
**characterized in that**
said computer (20) further comprises:
an assessing means (30) for calculating image data such that the gait status of the subject can be presented as an image in the form of a foot at each sampling time, based on estimation by said estimating means (22), and
a presentation means (31) for presenting a visualized gait of the subject on the ground or floor based on the image data calculated by said assessing means (30).

2. The gait assessment system according to claim 1, wherein said basograph (10) includes an acceleration sensor (13) as a part of the measuring instrument, the acceleration sensor (13) measuring, as the gait-related data, predetermined-direction acceleration of the subject's foot at each predetermined time, and
said estimating means (22) includes at least one of the following means for performing estimation based on the predetermined-direction acceleration of the subject's foot at each predetermined time: a push-off estimating means (23) for estimating the intensity of the subject's push-off; a tempo estimating means (24) for estimating the subject's gait tempo; an elevation estimating means (25) for estimating elevation of the subject's foot; and a stride estimating means (26) for estimating the subject's stride.

3. The gait assessment system according to claim 1 or 2, wherein said basograph (10) further includes an inclination sensor (14) as a part of the measuring instrument, the inclination sensor (14) measuring, as the gait-related data, a predetermined-direction inclination of the subject's foot at each predetermined time, and
said estimating means (22) further includes a landing estimating means (27) for estimating a landing status of the subject's foot based on the predetermined-direction acceleration of the subject's foot at each predetermined time, and the predetermined-direction inclination of the subject's foot at each predetermined time.

4. The gait assessment system according to any of claims 1 through 3, wherein said basograph (10) further includes an angular velocity sensor (15) as a part of measuring instrument, the angular velocity sensor (15) measuring, as the gait-related data, an angular velocity of the subject's foot at each predetermined time, and
said estimating means (22) further includes a moving-status estimating means (28) for estimating a moving status of the subject based on the predetermined-direction acceleration of the subject's foot at each predetermined time, and the angular velocity of the subject's foot at each predetermined time.

5. The gait assessment system according to any of claims 1 through 4, wherein said basograph (10) further includes an operating means (16) for performing a predetermined operation based on the gait-related data measured by the measuring instrument.

## Patentansprüche

1. Gangzugriffssystem, das umfasst:
ein Messinstrument (12) zum Messen gangbezogener Daten einer Testperson, einen Basographen bzw. ein Gerät zur Aufzeichnung von Fußabdrücken (10), das mit dem Messinstrument ausgestattet ist, und einen Computer (20), der fähig ist, die von dem Messinstrument gemessenen gangbezogenen Daten durch ein vorgegebenes Empfangsverfahren zu empfangen,
wobei der Computer (20) umfasst:
ein Bewertungsmittel (22) zum Bewerten eines Gangzustands der Testperson basierend auf den empfangenen gangbezogenen Daten,
**dadurch gekennzeichnet, dass**
der Computer (20) ferner umfasst:
ein Zugriffsmittel (30) zum Berechnen von Bilddaten, so dass der Gangzustand der Testperson basierend auf der Bewertung durch das Bewertungsmittel (22) als ein Bild in der Form eines Fußes zu jeder Abtastzeit dargestellt werden kann, und
ein Darstellungsmittel (31) zum Darstellen eines visualisierten Gangs der Testperson auf der Erde oder dem Boden basierend auf den von dem Zugriffsmittel (30) berechneten Bilddaten.

2. Gangzugriffssystem nach Anspruch 1, wobei der Basograph (10) einen Beschleunigungssensor (13) als einen Teil des Messinstruments umfasst, wobei der Beschleunigungssensor (13) als die gangbezogenen Daten zu jeder vorgegebenen Zeit die Beschleunigung des Fußes der Testperson in eine vorgegebene Richtung misst, und
das Bewertungsmittel (22) wenigstens eines der folgenden Mittel zum Durchführen der Bewertung basierend auf der Beschleunigung des Fußes der Testperson in einer vorgegebenen Richtung zu jeder vorgegebenen Zeit umfasst: ein Abstoßbewertungsmittel (23) zum Bewerten der Intensität des Abstoßens der Testperson; ein Geschwindigkeitsbewertungsmittel (24) zum Bewerten des Schritttempos der Testperson; ein Erhöhungsbewertungsmittel (25) zum Bewerten der Erhöhung des Fußes der Testperson; und ein Schrittbewertungsmittel (26) zum Bewerten des Schritts der Testperson.

3. Gangzugriffssystem nach Anspruch 1 oder 2, wobei der Basograph (10) ferner einen Neigungssensor (14) als einen Teil des Messinstruments umfasst, wobei der Neigungssensor (14) als die gangbezogenen Daten zu jeder vorgegebenen Zeit eine Neigung in einer vorgegebenen Richtung des Fußes der Testperson misst, und
das Bewertungsmittel (22) ferner ein Aufsetzbewertungsmittel (27) zum Bewerten eines Aufsetzzustands des Fußes der Testperson basierend auf der Beschleunigung des Fußes der Testperson in einer vorgegebenen Richtung zu jeder vorgegebenen Zeit, und der Neigung in der vorgegebenen Richtung des Fußes der Testperson zu jeder vorgegebenen Zeit umfasst.

4. Gangzugriffssystem nach einem der Ansprüche 1 bis 3, wobei der Basograph (10) ferner einen Winkelgeschwindigkeitssensor (15) als einen Teil des Messinstruments umfasst, wobei der Winkelgeschwindigkeitssensor (15) als die gangbezogenen Daten eine Winkelgeschwindigkeit des Fußes der Testperson zu jeder vorgegebenen Zeit umfasst,
wobei das Bewertungsmittel (22) ferner ein Bewegungszustandsbewertungsmittel (28) zum Bewerten eines Bewegungszustands der Testperson basierend auf der Beschleunigung in der vorgegebenen Richtung des Fußes der Testperson zu jeder vorgegebenen Zeit und der Winkelgeschwindigkeit des Fußes der Testperson zu jeder vorgegebenen Zeit umfasst.

5. Gangzugriffssystem nach einem der Ansprüche 1 bis 4, wobei der Basograph (10) ferner ein Bedienmittel (16) zum Durchführen einer vorgegebenen Bedienung basierend auf den von dem Messinstrument gemessenen gangbezogenen Daten umfasst.

## Revendications

1. Système d'évaluation de la démarche, comprenant :
un instrument de mesure (12) pour mesurer les données relatives à la démarche d'un sujet, un basographe (10) équipé de l'instrument de mesure et un ordinateur (20) capable de recevoir les données relatives à la démarche et mesurées par l'instrument de mesure par l'intermédiaire d'une méthode de réception prédéterminée,
dans lequel ledit ordinateur (20) inclut :
un moyen d'estimation (22) pour estimer un état de la démarche du sujet sur la base des données reçues et relatives à la démarche,
**caractérisé en ce que**
lequel ledit ordinateur (20) comprend en outre :
un moyen d'évaluation (30) pour le calcul des données d'image de telle sorte que l'état de la démarche du sujet peut être présenté comme une image sous la forme d'un pied à chaque moment d'échantillonnage, sur la base d'une estimation par ledit moyen d'estimation (22), et
un moyen de présentation (31) pour présenter une démarche visualisée du sujet sur la terre ou sur le sol sur la base des données d'image calculées par ledit moyen d'évaluation (30).

2. Système d'évaluation de la démarche conformément à la revendication 1, dans lequel ledit basographe (10) inclut un capteur d'accélération (13) comme une partie de l'instrument de mesure, dans lequel le capteur d'accélération (13) mesure, en tant que données relatives à la démarche, l'accélération de direction prédéterminée du pied de sujet à chaque moment prédéterminé, et
ledit moyen d'estimation (22) inclut au moins un parmi les moyens suivants pour accomplir l'estimation sur la base de l'accélération de direction prédéterminée à chaque moment prédéterminé : un moyen d'estimation de recul (23) pour estimer l'intensité du recul de sujet ; un moyen d'estimation de la vitesse (24) pour estimer la vitesse de démarche du sujet ; un moyen d'estimation de l'élévation (25) pour estimer l'élévation du pied de sujet ; et un moyen d'estimation de l'enjambée (26) pour estimer l'enjambée du sujet.

3. Système d'évaluation de la démarche conformément à l'une ou l'autre des revendications 1 et 2, dans lequel ledit basographe (10) inclut en outre un capteur d'inclinaison (14) comme une partie de l'instrument de mesure, dans lequel le capteur d'inclinaison (14) mesure, en tant que données relatives à la démarche, une inclinaison de direction prédéterminée du pied de sujet à chaque moment prédéterminé, et
ledit moyen d'estimation (22) inclut en outre un moyen d'estimation de pose (27) pour estimer un état de pose du pied de sujet sur la base de l'accélération de direction prédéterminée du pied de sujet à chaque moment prédéterminé et de l'inclinaison de direction prédéterminée du pied de sujet à chaque moment prédéterminé.

4. Système d'évaluation de la démarche conformément à l'une quelconque des revendications 1 à 3, dans lequel ledit basographe (10) inclut en outre un capteur de vitesse angulaire (15) comme une partie de l'instrument de mesure, dans lequel le capteur de vitesse angulaire (15) mesure, en tant que données relatives à la démarche, une vitesse angulaire du pied de sujet à chaque moment prédéterminé, et
ledit moyen d'estimation (22) inclut en outre un moyen d'estimation de l'état de mouvement (28) pour estimer un état de mouvement du sujet sur la base de l'accélération de direction prédéterminée du pied de sujet à chaque moment prédéterminé et de la vitesse angulaire du pied de sujet à chaque moment prédéterminé.

5. Système d'évaluation de la démarche conformément à l'une quelconque des revendications 1 à 4, dans lequel ledit basographe (10) inclut en outre un moyen de commande (16) pour accomplir une commande prédéterminée sur la base des données relatives à la démarche et mesurées par l'instrument de mesure.
